# EUROPEAN PATENT APPLICATION

(11) **EP 0 572 975 A1**
(43) Date of publication of application: **08.12.1993**
(21) Application number: 93108797.7
(22) Date of filing: 01.06.1993
(51) Int. Cl.: A61L 2/18, A61L 2/10, C02F 1/32, C02F 1/72

(54) **Fluid sterilizing process and device**

(30) Priority: 02.06.1992 IT TO920468
(71) Applicant: U.V.T. SUD S.r.l. ULTRAVIOLET TECHNOLOGY SUD, I-74027 San Giorgio Jonico (IT)
(72) Inventor: d'Alessandro, Vito Antonio, I-74100 Taranto (IT); Angelini, Pasquale, I-74100 Taranto (IT)
(74) Representative: Plebani, Rinaldo

(57) **Abstract**

A process and device (1; 34) whereby a fluid, even a fluid impervious to ultraviolet rays, is fed along a conduit (8) consisting at least partly of non-wettable material transparent to ultraviolet rays. The conduit (8) extends through a casing (2) housing at least one ultraviolet-ray source (24, 25, 26) located outside, and in front of the transparent part of, the conduit (8). Substantially at the inlet pipe (14) of the conduit (8), a peroxide solution (31), the decomposition of which is catalyzable by the ultraviolet rays, is fed into the fluid flowing in the conduit (8), and is mixed with the fluid and fed, together with the fluid and inside the conduit (8), through the casing (2) housing the ultraviolet-ray source (24, 25, 26).

## Description

The present invention relates to a fluid sterilizing process, and to a device implementing such a process.

Ultraviolet rays have long been known to be highly effective as a means of sterilizing fluids.

In the past, this property of ultraviolet rays has normally been employed for sterilizing water or other alimentary fluids. In particular, purifying and sterilizing devices for drinks, such as water or similar and transparent to ultraviolet rays, are employed wherein the drink is fed through at least a vessel or a tubular conduit defined by a wall at least partly transparent to ultraviolet rays and located in front of at least one ultraviolet ray source. The ultraviolet rays produced by the source are thus directed through the transparent wall of the conduit and the drink, which is also transparent, so as to bombard the particles of the drink in depth and so effectively destroy any bacteria.

A major drawback of purifying and sterilizing devices of the aforementioned type is that they are unsuitable for sterilizing fluids other than drinks, such as swimming pool and/or bathing water, which may contain organic pollutants which are in no way affected by ultraviolet rays.

Such organic pollutants are normally degraded by oxidation, that is, by adding to the fluid an oxidizing substance which, in certain applications, must also be harmless to the human organism. For swimming pool and bathing water, for example, chlorine is generally added (so-called "chlorination" process), which, though harmless to the user and relatively effective at normal concentrations, results in an unpleasant taste and odour of the water, and, in the case of highly sensitive users, may also result in irritation and inflammation, particularly of the mucous membrane and eyes.

It is an object of the present invention to provide a fluid sterilizing process designed to overcome the aforementioned drawbacks.

According to the present invention, there is provided a fluid sterilizing process comprising stages consisting in:
- feeding particles of the fluid along a conduit consisting at least partly of material transparent to ultraviolet rays; and
- simultaneously bombarding said particles flowing along said conduit with ultraviolet rays generated by at least one source outside said conduit;
   characterized by the fact that, inside said conduit, the particles of said fluid are mixed with a peroxide solution, the decomposition of which is catalyzable by the ultraviolet rays, and which is fed into the conduit in a predetermined amount from a tank.

According to the above process, a stream of hydrogen peroxide (H₂O₂) is fed into the conduit and mixed therein with the fluid; on being bombarded with ultraviolet rays, the hydrogen peroxide decomposes, giving rise to an oxidation-reduction reaction wherein the peroxide is simultaneously the oxidant and the reducer, and which produces in the fluid nascent gaseous oxygen, which in turn oxidizes the organic pollutants in the fluid.

Said conduit preferably comprises a thin passage defined by two walls, at least one of which consists of material transparent to ultraviolet rays, and at least one of which consists of non-wettable material designed to produce, in the fluid flowing inside the passage, microvortices of substantially the same diameter as the thickness of the passage; the peroxide solution being fed into the conduit upstream from the passage, so that the ultraviolet rays produced by said source simultaneously bombard both the peroxide solution and the particles flowing along the passage, as they swirl along the passage and are mixed and brought substantially into contact with said transparent wall.

As such, sterilization is no longer effected by the ultraviolet rays penetrating the fluid, but by bombarding the fluid superficially, thus overcoming the functional limitation posed by the fluid necessarily being transparent.

According to the present invention, there is also provided a fluid sterilizing device comprising a conduit for a stream of particles of the fluid to be sterilized, said conduit consisting at least partly of material transparent to ultraviolet rays; and at least one ultraviolet ray source positioned facing the transparent portion of said conduit; characterized by the fact that it also comprises means for supplying a peroxide solution, the decomposition of which is catalyzable by the ultraviolet rays, from a solution storage tank to a point along said conduit.

The present invention will be described with reference to the accompanying drawings, which show axial sections of two different non-limiting embodiments.

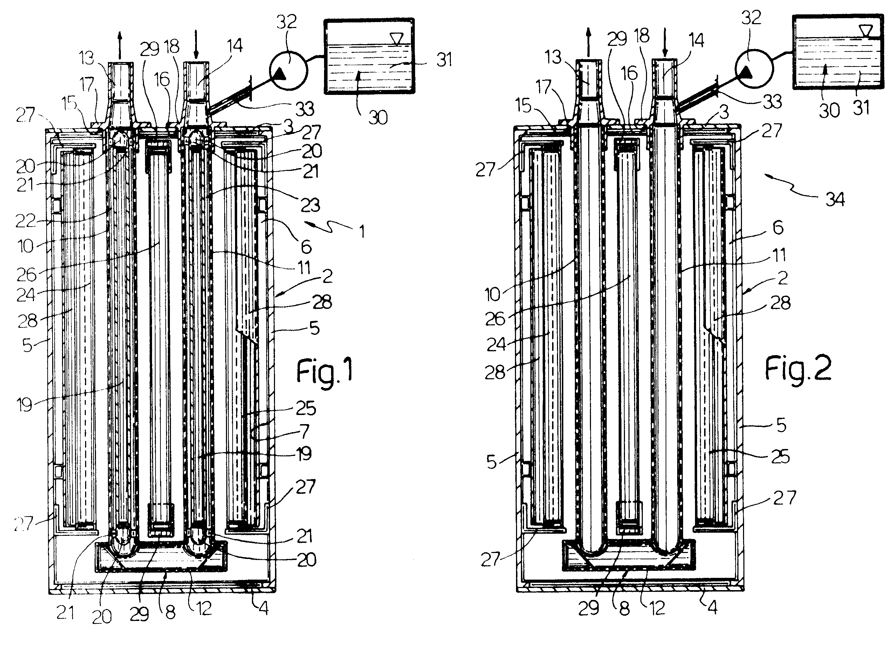

Number 1 in Fig.1 indicates a purifying and sterilizing device for fluids, even fluids impervious to ultraviolet rays, comprising an outer casing 2 preferably made of metal and in the form of a rectangular parallelepipedon.

The casing 2 shown comprises a top wall 3; a bottom wall 4; a first pair of parallel lateral walls 5; and a second pair of parallel lateral walls 6 (only one shown) perpendicular to walls 5.

The chamber 7 defined by walls 3, 4, 5 and 6 houses a fluid conduit 8, which may be any shape and, in the example shown, is substantially U-shaped with its concavity facing upwards. More specifically, conduit 8 comprises an upflow conduit 10 and a downflow conduit 11 parallel to each other, consisting of non-wettable material transparent to ultraviolet rays, and connected to each other at the bottom end by a horizontal conduit 12. The top ends of conduits 10 and 11 are engaged inside respective tubular fittings of respective outlet and inlet pipes 13 and 14 extending through respective holes 15 and 16 in wall 3 and connected to wall 3 by means of respective flanges 17 and 18.

Each conduit 10, 11 houses a tubular, preferably metal element 19 closed at each end by caps 20, each of which is fitted to the inner surface of respective conduit 10, 11 by means of radial tabs 21.

Tubular elements 19 extend along substantially the whole length of respective conduits 10, 11, and define, with the same, respective toroidal passages 22, 23 along which the fluid to be sterilized is fed.

In the example shown, chamber 7 is fitted inside with three fluorescent ultraviolet-ray lamps 24, 25, 26. Lamps 24 and 25 are connected by respective end brackets 27 to respective walls 5, adjacent to respective conduits 10 and 11, and present respective paraboloidal reflectors 28 for concentrating the emitted rays on to conduits 10 and 11; and lamp 26 is positioned between conduits 10 and 11, and is supported by end brackets 29 fitted to one of walls 6.

According to the present invention, device 1 also comprises a storage tank 30 for a peroxide solution 31, decomposition of which is catalyzable by the ultraviolet rays. Tank 30, which for the sake of simplicity is shown only schematically, may be of any shape, and is preferably fitted to the outside of casing 2, e.g. supported in known manner (by means of brackets not shown) on one of walls 3, 4, 5 or 6. Tank 30 presents a known metering pump 32, also shown only schematically, for supplying peroxide solution 31 to a point along conduit 8.

In particular, pump 32 feeds solution 31 directly into inlet pipe 14 along a lateral pipe 33, immediately upstream from passage 23 of downflow conduit 11 and outside casing 2. Peroxide solution 31 consists of stabilized hydrogen peroxide of a predetermined concentration.

In actual use, the fluid to be sterilized is fed from the mains (not shown) to inlet pipe 14, and successively along passage 23, conduit 12 and passage 22 of conduit 8 to outlet pipe 13.

As it flows along toroidal passages 22 and 23, the fluid to be sterilized assumes a swirling motion due to the fact that, as conduits 10 and 11 are made of non-wettable material, such as polytetrafluoroethylene, the fluid particles substantially contacting the inner surface of conduits 10 and 11 encounter substantially no frictional resistance and so tend to travel faster than the other particles. The size of the vortices formed inside passages 22 and 23 remains substantially constant for a given flow speed, so that, by selecting the thickness of passages 22 and 23 so that it roughly equals the diameter of the vortices produced at the required flow speed, it is possible to bring all the fluid particles successively into contact with the inner surfaces of conduits 10 and 11, that is, into a position in which the particles can be bombarded superficially and so sterilized by the ultraviolet rays emitted by lamps 24, 25 and 26.

According to the present invention, the sterilizing action of the ultraviolet rays is also accompanied by strong chemical oxidation. In fact, simultaneously with the fluid to be sterilized, a predetermined amount of peroxide solution 31 is fed by pump 32 from tank 30 and along lateral pipe 33 into pipe 14, i.e. upstream from the portion of conduit 8 exposed to the ultraviolet rays produced by lamps 24, 25 and 26. On entering the fluid flow, peroxide solution 31, hydrogen peroxide in the example shown, is mixed closely with the fluid by virtue of the dispersive action of said swirling motion to which the fluid is subjected as it flows along passages 23 and 22. Moreover, the action of said vortices also brings all the hydrogen peroxide particles into contact with the inner surfaces of conduits 10 and 11, i.e. into a position in which, like the fluid particles, they may be superficially bombarded by the ultraviolet rays emitted by lamps 24, 25 and 26.

As such, decomposition of the hydrogen peroxide particles is catalyzed (in particular, photocatalyzed) to produce nascent gaseous oxygen in the fluid flowing along conduit 8, and so oxidize any organic pollutant substances contained in the fluid. The oxidizing action of the gaseous oxygen is also accompanied by further oxidizing action and the reducing action of the hydrogen peroxide on direct contact with the pollutant substances. In fact, under the conditions described of the process according to the present invention, the hydrogen peroxide is located in the intermediate portion of the Pourbaix diagram, wherein it acts both as an oxidant and a reducer. Finally, at least a small part of the oxygen produced by decomposition of the hydrogen peroxide may be converted by the ultraviolet rays into ozone, which, under process conditions, is restored immediately to the normal atomic state (oxygen), but not before being brought into contact by said swirling motion with any organic particles in the fluid, which are thus strongly oxidized.

By the time the fluid reaches outlet pipe 13, all the hydrogen peroxide fed initially into pipe 14 is decomposed into water and oxygen, according to the following well known reaction:

(1) 2 H₂O₂ --> 2 H₂O + O₂

so that the sterilized fluid at the outlet no longer contains any peroxide, by virtue of the equilibrium of reaction (1) under the process conditions described being shifted completely to the left. Similarly, any organic pollutant substances initially contained in the fluid are completely destroyed by the chemical action produced in device 1 by feeding hydrogen peroxide into conduit 8. As such, the sterilized, purified fluid at the outlet of device 1 is perfectly safe healthwise, and, at the same time, is free of the unpleasant odour and taste typically associated with fluids sterilized using chlorine.

Number 34 in Fig.2 indicates a simplified version of device 1, which may be used for fluids transparent to ultraviolet rays. For the sake of simplicity, any details similar or identical to those described with reference to device 1 are shown using the same numbering system.

In particular, device 34 is identical to device 1, except for the absence of tubular elements 19 and respective caps 20 and radial tabs 21 for fitting them to conduits 10 and 11. Conduit 8 thus presents no passages 22, 23, and the fluid for sterilizing flows along the entire section of conduits 10 and 11. In this case, the swirling motion of the fluid along conduit 8 is produced solely by the travelling speed of the fluid, but is nevertheless sufficient to enable hydrogen peroxide fed into inlet pipe 14 by pump 32 to mix with the fluid, in exactly the same way as on device 1. To sterilize the fluid particles and photocatalyze decomposition of the hydrogen peroxide particles, the particles need not contact the inner wall of conduits 10 and 11, as on device 1, by virtue of the fluid being transparent to the ultraviolet rays, which may thus penetrate the entire cross section of conduits 10 and 11 and so directly reach all the fluid and hydrogen peroxide particles flowing along conduit 8. As for the rest, operation and the end results of device 34 are identical to those of device 1.

To those skilled in the art it will be clear that changes may be made to devices 1 and 34 as described and illustrated herein without, however, departing from the scope of the present invention.

For example, according to a first variation (not shown), conduit 8 may consist of a coil, or be of any appropriate shape other than that described herein.

According to a further variation (not shown) of device 1, passages 22 and 23 may be flat as opposed to toroidal as in the example shown; and tubular elements 19 may be made of material capable of reflecting ultraviolet rays, or themselves be made of non-wettable material transparent to ultraviolet rays.

According to a further variation (not shown), the ultraviolet-ray lamps may differ as to number and/or location.

Finally, according to a further variation (not shown), devices 1 and 34 may constitute a sterilizing unit in a system comprising a number of such units connected to one another in various ways and housed inside respective casings 2 or inside a single casing.

## Claims

1. A fluid sterilizing process comprising stages consisting in:
- feeding particles of the fluid along a conduit (8) consisting at least partly of material transparent to ultraviolet rays; and
- simultaneously bombarding said particles flowing along said conduit (8) with ultraviolet rays generated by at least one source (24, 25, 26) outside said conduit (8); characterized by the fact that, inside said conduit (8), the particles of said fluid are mixed with a peroxide solution (31), the decomposition of which is catalyzable by the ultraviolet rays, and which is fed into the conduit (8) in a predetermined amount from a tank (30).

2. A process as claimed in Claim 1, characterized by the fact that said conduit (8) comprises a thin passage (22, 23) defined by two walls (10, 11 and 19), at least one of which is made of material transparent to ultraviolet rays, and at least one of which is made of non-wettable material designed to produce, in the fluid flowing in said passage (22, 23), microvortices of substantially the same diameter as the thickness of said passage; said peroxide solution (31) being fed into said conduit (8) upstream from said passage (22, 23), so that the ultraviolet rays produced by said source (24, 25, 26) simultaneously bombard both the peroxide solution (31) and said particles flowing along said passage (22, 23), as they swirl along said passage and are mixed and brought substantially into contact with said transparent wall (10, 11).

3. A process as claimed in Claim 1 or 2, characterized by the fact that a stream of hydrogen peroxide (31) is fed into said conduit (8) and mixed therein with the fluid for sterilizing; the hydrogen peroxide, when bombarded with the ultraviolet rays, being decomposed to give rise to an oxidation-reduction reaction wherein the peroxide acts as both an oxidant and a reducer, and which produces nascent gaseous oxygen in the fluid for sterilizing; the oxygen so formed in turn oxidizing any organic pollutant substances contained in the fluid.

4. A fluid sterilizing device (1; 34) comprising a conduit (8) for a stream of particles of the fluid to be sterilized, said conduit (8) consisting at least partly of material transparent to ultraviolet rays; and at least one ultraviolet ray source (24, 25, 26) positioned facing the transparent portion of said conduit (8); characterized by the fact that it also comprises means (32, 33) for supplying a peroxide solution (31), the decomposition of which is catalyzable by the ultraviolet rays, from a solution storage tank (30) to a point along said conduit (8).

5. A device (1) as claimed in Claim 4, characterized by the fact that said conduit (8) comprises two walls (10, 11 and 19) facing each other and defining a relatively thin passage (22, 23); at least one of said walls (10, 11) consisting at least partly of material transparent to ultraviolet rays, and at least one of said walls (19) consisting of non-wettable material; said feed means (32, 33) being connected to a point of said conduit (8) upstream from said passage (22, 23); and the thickness of said passage being roughly equal to the diameter of vortices produced, in use, inside said stream by said wall (19) of non-wettable material.

6. A device (1) as claimed in Claim 5, characterized by the fact that said passage (22, 23) is toroidal in shape; said two walls (10, 11, 19) being coaxial tubular walls.

7. A device (1) as claimed in Claim 6, characterized by the fact that a first (10, 11) of said tubular walls, located externally, consists of said non-wettable material transparent to ultraviolet rays; said ultraviolet-ray source (24, 25, 26) being located outside said outer wall.

8. A device (1; 34) as claimed in any one of the foregoing Claims from 4 to 7, characterized by the fact that said feed means comprise a metering pump (32) for withdrawing a predetermined amount of said peroxide solution (31) from said tank (30) and feeding it into the inlet pipe (14) of said conduit (8) along a lateral pipe (33) outside a casing (2) fitted through with said conduit (8) and housing said ultraviolet-ray source (24, 25, 26); said storage tank (30) being fitted to the outside of said casing (2).
